# EUROPEAN PATENT APPLICATION

(11) **EP 1 970 707 A1**
(43) Date of publication of application: **17.09.2008**
(21) Application number: 06843270.7
(22) Date of filing: 26.12.2006
(51) Int. Cl.: G01N 33/569, C07K 16/10, G01N 33/543, C12N 15/09, C12P 21/08, G01N 33/577

(54) **METHOD FOR DETECTION OF VIRULENT STRAIN OF INFLUENZA TYPE-A VIRUS**

(30) Priority: 26.12.2005 JP 2005373584
(71) Applicant: BL Co., Ltd, Numazu-shi, Shizuoka 410-0042 (JP)
(72) Inventor: NAMBA, Yasuharu, Numazu-shi, Shizuoka 4100042 (JP)
(74) Representative: Eidelsberg, Victor Albert
(86) International application number: PCT/JP2006/325886
(87) International publication number: WO 2007/074811

(57) **Abstract**

The present invention provides a method for detecting a virulent strain of influenza virus in a specific, rapid and simple manner, and an assay device therefor. A method for detecting a virulent strain of influenza A virus is provided, which comprises providing a first antibody that is reactive with all influenza A virus subtypes and a second antibody that is not reactive with a virulent strain of influenza A virus but is reactive with all influenza A virus subtypes other than the virulent strain, and conducting an immunoassay to detect an antigen that shows a positive response in a reaction with the first antibody and shows a negative response in a reaction with the second antibody.

## Description

### TECHNICAL FIELD

The present invention relates to a method for detection of a virulent strain of influenza A virus using an antibody reactive with all influenza A virus subtypes other than the virulent strain of influenza A virus, and more specifically relates to a sandwich immunoassay, and particularly an immunochromatographic assay and an immunochromatographic assay device. The present invention relates to a detection method useful for a rapid and simple diagnosis of infection with a virulent strain of influenza viruses such as a highly pathogenic avian influenza virus.

### BACKGROUND ART

Highly pathogenic avian influenza is an infectious disease caused by an influenza virus that exhibits deadly pathogenicity to chickens and the like. This disease is also called fowl plague. In Japan, this disease is an infectious disease legally-designated under the domestic animal infectious disease prevention law. Also, highly pathogenic avian influenza is designated as a List A disease by the Office International des Epizooties (OIE).

The influenza viruses that have caused such highly pathogenic avian influenza fall under influenza A virus subtypes H5 and H7 only. These subtypes are not necessarily virulent. However, when domestic animals are infected with these subtypes, all the animals must be killed in Japan whether they are virulent or attenuated.

With regard to human infection with avian influenza viruses, infection with avian influenza virus subtypes H5, H7 and H9 has been reported. Of these, the H5N1 subtype is the only influenza virus that has been reported to cause patients' death. It has been reported that infection with the influenza virus subtype H5N1 causes serious pneumonia followed by death.
In 1997, highly pathogenic avian influenza caused by the subtype H5N1 has become epidemic in Hong Kong. Further, this has become so epidemic in several Asian countries after 2003 until now, and even human infection and death have been reported.
At present, the subtype H5N1 is of great concern about human infection over the world, and is watched causing a new influenza following the subtypes H1N1 and H3N2 in the world.

According to the WHO report of November 17, 2005, there were 130 cases in which human infection with the influenza virus subtype H5N1 was confirmed, and among them, there were 67 cases in which patients died. After December 2004, the number of cases in which the human infection was confirmed has rapidly increased, and death toll has also increased. The mortality rate of patients after being infected with the H5N1 virus is 51.3% (67/130).
As is clear from the above descriptions, human infection with the subtype H5N1 has now become a reality. In order to prevent a large-scale infection with influenza, rapid detection of infection with the subtype H5N1 is an important issue. In addition, rapid diagnosis is also important for treatment thereof.

Under the current circumstances, diagnosis of highly pathogenic avian influenza for birds is carried out by a method which comprises collecting a tracheal swab or a cloaca swab (cloacal swab) from a sick bird suspected of virus infection, inoculating it in an embryonated chicken egg for culture, and then separating the virus. However, this method is disadvantageous in that it takes several days to obtain the results and thus the results cannot be rapidly obtained. Using recently developed genetic tests (PCR method, LAMP method), the time required for obtaining the results is significantly reduced. However, since these genetic tests require special equipment and techniques, it is impossible to carry out these tests in a poultry farm and the like.

Influenza virus is infected through human upper respiratory tract, lower respiratory tract, or the like, and after an incubation period of 1 to 2 days, the virus causes headache, low back pain, myalgia, general malaise, disorder of digestive organs, etc., as well as fever (38°C to 39°C). The most problematic complications of such influenza include pneumonia in aged people and encephalitis/encephalopathy in children. In particular, encephalitis/encephalopathy in children is characteristic in that it is attended with high fever, consciousness disorder, and convulsion. Prognosis of such encephalitis/encephalopathy in children is extremely poor, and the period from initial symptoms to expression of central nervous system symptoms or death is extremely short. Thus, rapid diagnosis and treatment are necessary.

Previously, people were diagnosed as influenza-like disease based on the aforementioned clinical symptoms only. In recent years, however, as a result of development and wide spread of test kits for rapidly detecting influenza virus antigens, it has become possible to diagnose influenza virus infectious disease at an early stage. Such rapid diagnostic kits include those utilizing enzyme immunoassay (EIA) or immunochromatographic assay as a principle. Some kits detect only influenza A virus, and other kits detect influenza A and B viruses collectively. Further, some other kits detect influenza A and B viruses individually.
However, the current influenza antigen tests employ an antibody that is reactive with a nucleoprotein common to all the A viruses. Thus, differential diagnosis between infections with the subtype H5 and the subtype H1N1 or H3N2, all of which are influenza A viruses, cannot be carried out.

The subtype H5N1 infection causes serious pneumonia, and is extremely high in mortality rate. Thus, it is important to conduct the differential diagnosis of the subtype H5 infection at the time of initial diagnosis, which will also be useful to determine a therapeutic strategy for the patient. This also makes it possible to take measures such as segregation of the patient at the early stage so as to prevent further infection, which will be important to prevent further expansion of the subtype H5N1 infection.
Using the recently developed genetic tests (PCR method, LAMP method), it is possible to differentiate and detect the subtype H5N1. However, these genetic tests require special equipment and techniques, and thus it cannot be said that they are simple methods.

Patent Document 1: Japanese Patent Laid-open (Kohyo) No. 2004-509648
Patent Document 2: Japanese Patent Laid-Open (Kokai) No. 11-108932
Patent Document 3: Japanese Patent Laid-Open (Kokai) No. 2001-215228
Non-Patent Document 1: Hinshaw V.S. et al., "Specific antibody responses and generation of antigenic variants in chickens immunized against a virulent avian influenza virus," Avian Dis. 1990, 34(1): 80-6
Non-Patent Document 2: Kaverin N.V. et al., "Structure of antigenic sites on the haemagglutinin molecule of H5 avian influenza virus and phenotypic variation escape mutants," J. Gen. Virol. 2002, 83(pt10): 2497-505

### DISCLOSURE OF THE INVENTION

### PROBLEM TO BE SOLVED BY THE INVENTION

As mentioned above, influenza viruses rapidly infect, and quickly spread in a wide range if the detection is delayed. Thus, it is necessary to detect influenza virus infection as early and quickly as possible. In particular, the influenza A virus subtype H5N1 causes serious pneumonia, and is extremely high in mortality rate. Nevertheless, up to now, it was impossible to detect the influenza A virus subtype H5 separately from other types of influenza A virus infections by use of conventional reagents for detecting influenza virus antigens.
It is an object of the present invention to provide a detection reagent that enables a rapid and simple differential diagnosis of a virulent strain of influenza A virus including an influenza A virus subtype H5N1, and detection methods using the same, including a sandwich immunoassay, and in particular, an immunochromatographic assay, and an immunochromatographic assay device.

### MEANS FOR SOLVING THE PROBLEM

The present inventor has obtained various types of antibodies by immunizing mice with influenza A virus as an immunogen. Then, these antibodies have been tested in immunochromatographic assay devices. During this process, the inventor has succeeded in obtaining a monoclonal antibody reactive with all influenza A virus subtypes other than a virulent strain of influenza virus. Then, the present invention has been completed by finding out that a virulent strain of influenza A virus can be detected using the aforementioned antibody in an immunoassay, particularly in a sandwich immunoassay, and specifically an immunochromatographic assay.

That is to say, in accordance with one aspect of the present invention, there is provided a method for detecting a virulent strain of influenza A virus, which comprises providing a first antibody that is reactive with all influenza A virus subtypes and a second antibody that is not reactive with a virulent strain of influenza A virus but is reactive with all influenza A virus subtypes other than the virulent strain, and conducting an immunoassay to detect an antigen that shows a positive response in a reaction with the first antibody and shows a negative response in a reaction with the second antibody.

The immunoassay used in this detecting method is not particularly limited. A sandwich immunoassay, particularly, an ELISA (Enzyme-linked immunosorbent assay) method, an immunochromatographic assay, and the like are preferable.
Thus, in a preferred embodiment of the aforementioned detecting method of the present invention, the aforementioned immunoassay comprises a sandwich immunoassay using the first antibody and a third antibody that is reactive with an antigen with which the first antibody is reactive, and/or a sandwich immunoassay using the second antibody and a fourth antibody that is reactive with an antigen with which the second antibody is reactive. Such a sandwich immunoassay can advantageously be carried out by immobilizing the first antibody and the second or fourth antibody in a carrier. In this case, if a membrane carrier is used as a carrier, an immunochromatographic assay can be carried out.

Accordingly, in accordance with another aspect of the present invention, there is provided an immunochromatographic assay which comprises:
providing a membrane carrier having a first capturing zone and a second capturing zone which are formed in predetermined positions thereof by immobilizing a first antibody and a second antibody respectively, the first antibody being reactive with all influenza A virus subtypes, and the second antibody being not reactive with a virulent strain of influenza A virus but being reactive with all influenza A virus subtypes other than the virulent strain; and
chromatographically developing a first mixed solution and a second mixed solution in the membrane carrier toward the first capturing zone and the second capturing zone respectively, the first mixed solution containing a test sample and a third antibody reactive with an antigen with which the first antibody is reactive, and the second mixed solution containing the test sample and a fourth antibody reactive with an antigen with which the second antibody is reactive, or chromatographically developing, in the membrane carrier, the first mixed solution toward both the capturing zones,
whereby a virulent strain of influenza A virus can be detected based on a result that a positive response in a reaction with the first antibody is shown in the first capturing zone whilst a negative response in a reaction with the second antibody is shown in the second capturing zone.

In addition, in accordance with a further aspect of the present invention, there is provided an immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, a third antibody that is reactive with an antigen with which the first antibody is reactive, a fourth antibody that is reactive with an antigen with which the second antibody is reactive, and a membrane carrier, wherein the first and second antibodies are previously immobilized in predetermined positions of the membrane carrier so as to form a first capturing zone and a second capturing zone, and the third and fourth antibodies are labeled with suitable labeling agents, and wherein the third and fourth antibodies are prepared at a position remote from the capturing zones for being chromatographically developed in the membrane carrier toward the first and second capturing zones, respectively.

The aforementioned membrane carrier may be a single membrane carrier having the first capturing zone and the second capturing zone that are apart from each other. Otherwise, the membrane carrier may comprise a first membrane carrier in which the first antibody is immobilized and a second membrane carrier in which the second antibody is immobilized. The latter membrane carrier is excellent in terms of specificity, and thus is preferable. In the case of the latter membrane carrier, it is convenient that the third antibody is prepared on the first membrane carrier and the fourth antibody is prepared on the second membrane carrier because measurement can be carried out only by injecting a test sample into each membrane carrier. It is convenient that the third and fourth antibodies are prepared whilst being impregnated into members disposed on the first and second membrane carriers, respectively.
When the membrane carrier of the present invention comprises the aforementioned first and second membrane carriers, they may be made into a so-called immunochromatographic test strip in which both the first and second membrane carriers have an elongated form, namely, a strip form, and the impregnated members disposed on the respective membrane carriers are placed at a certain distance from each other whilst the first and second membrane carriers are placed in parallel with each other. Alternatively, a so-called immunochromatographic test strip or immunochromatographic test kit can be formed in which both the first and second membrane carriers have an elongated form, namely, a strip form, and the impregnated members disposed on the respective membrane carriers are placed at a certain distance from each other whilst the first and second membrane carriers are placed radially or oppositely.

The aforementioned methods and devices all relate to a technique in which the aforementioned second antibody is immobilized in a carrier. In a preferred embodiment of the present invention, however, a technique in which the aforementioned second antibody is not immobilized in a carrier can also be adopted.
That is to say, in accordance with another aspect of the present invention, there is provided an immunochromatographic assay which comprises:
providing a first antibody that is reactive with all influenza A virus subtypes, a second antibody that is not reactive with a virulent strain of influenza A virus but is reactive with all influenza A virus subtypes other than the virulent strain, and a third antibody that is reactive with an antigen with which the first antibody is reactive;
preparing membrane carriers each having a first capturing zone which is formed in a predetermined position thereof by immobilizing the first antibody; and
chromatographically developing a first mixed solution and a second mixed solution in the respective membrane carriers toward the first capturing zone, the first mixed solution containing a test sample and the second antibody, and the second mixed solution containing the test sample and the third antibody,
whereby a virulent strain of influenza A virus can be detected based on a result that a positive response in a reaction with the third antibody is shown in the first capturing zone whilst a negative response in a reaction with the second antibody is shown in the first capturing. This assay requires only a single type of membrane carrier in which the first antibody is immobilized, but requires the chromatographic development twice, namely, the chromatographic development of the first mixed solution and the chromatographic development of the second mixed solution. Thus, this assay requires total two membrane carriers for the above two chromatographic developments.

Moreover, in accordance with another aspect of the present invention, there is provided an immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, a third antibody that is reactive with an antigen with which the first antibody is reactive, and a membrane carrier, wherein the first antibody is previously immobilized in a predetermined position of the membrane carrier so as to form a first capturing zone, and the second and third antibodies are labeled with suitable labeling agents, and wherein the second and third antibodies are each prepared at a position remote from the capturing zone for being chromatographically developed in the membrane carrier toward the first capturing zone.
In the aforementioned assay device, the membrane carrier preferably comprises at least a first membrane carrier in which the first antibody is immobilized and a second membrane carrier in which the first antibody is immobilized. It is convenient that the second antibody is prepared on the first membrane carrier and the third antibody is prepared on the second membrane carrier because measurement can be carried out only by injecting a test sample into the two membrane carriers. It is convenient that the second and third antibodies are prepared whilst being impregnated into members which are disposed on the first and second membrane carriers, respectively.

The detection method and assay of the present invention can be carried out by the combined use of a commercially available immunochromatographic test strip or kit for differential measurement of influenza A and B, such as CAPILIA (registered trademark) FluA+B (manufactured by Tauns Laboratories, Inc.), with a sandwich immunoassay device using the aforementioned first antibody and second antibody.
Accordingly, for such an embodiment, in accordance with another aspect of the present invention, there is provided an immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, and a membrane carrier, wherein the second antibody is previously immobilized in a predetermined position of the membrane carrier so as to form a first capturing zone, and the first antibody is labeled with a suitable labeling agent, and wherein the first antibody is prepared at a position remote from the first capturing zone for being chromatographically developed in the membrane carrier toward the first capturing zone.

Furthermore, for the same embodiment, in accordance with another aspect of the present invention, there is provided an immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, and a membrane carrier, wherein the first antibody is previously immobilized in a predetermined position of the membrane carrier so as to form a first capturing zone, and the second antibody is labeled with a suitable labeling agent, and wherein the second antibody is prepared at a position remote from the first capturing zone for being chromatographically developed in the membrane carrier toward the first capturing zone.

The aforementioned first antibody and second antibody used in the present invention are each an antibody against influenza viruses, and are generally obtained as antibodies against a nucleoprotein of influenza viruses. Each of the aforementioned first and second antibodies may be either a polyclonal antibody or a monoclonal antibody, and is preferably a monoclonal antibody from the viewpoint of specificity.
As the aforementioned first antibody, a known monoclonal antibody against a nucleoprotein of influenza A virus can generally be used.
The aforementioned second antibody has been obtained whilst various monoclonal antibodies against nucleoproteins of influenza A virus are examined for reactivity with various influenza A virus subtypes.

In the case of the first antibody, the aforementioned polyclonal antibody can be obtained, for example, by cloning a DNA fragment corresponding to a zone containing amino acid residues constituting an epitope from a DNA sequence encoding the amino acid sequence shown in SEQ ID NO: 6, allowing the cloned gene to express in a host such as *Escherichia coli* in a genetic engineering manner, extracting and purifying the expressed protein, immunizing an animal with the purified protein being used as an antigen according to an ordinary method, and then obtaining the polyclonal antibody from the antiserum of the immunized animal. In the case of the second antibody, the aforementioned polyclonal antibody can be obtained, for example, by cloning a DNA fragment corresponding to a zone containing amino acid residues constituting an epitope different from the influenza virus subtype H5N1 from a DNA sequence encoding the amino acid sequence shown in SEQ ID NO: 6, allowing the cloned gene to express in a host such as *Escherichia coli* in a genetic engineering manner, extracting and purifying the expressed protein, immunizing an animal with the purified protein being used as an antigen according to an ordinary method, and then obtaining the polyclonal antibody from the antiserum of the immunized animal.
In both cases of the first and second antibodies, a monoclonal antibody can be obtained, for example, by immunizing an animal such as a mouse with the above purified protein being used as an antigen, fusing the splenic cells of the immunized animal with myeloma cells for cell fusion, selecting the thus fused cells in a HAT-containing medium and allowing them to grow, and then selecting the grown strains using the above purified protein by an enzyme-labeled immunoassay or the like.

The monoclonal antibody used as the aforementioned second antibody is not reactive with a virulent strain of influenza A virus but is reactive with all influenza A virus subtypes other than the virulent strain. Such a monoclonal antibody, in particular, recognizes an epitope that is conserved in all influenza A virus subtypes other than a virulent strain of influenza virus and is mutated and lost in the virulent strain of influenza virus, and especially recognizes the epitope of a nucleoprotein of influenza A virus. It is considered that such an epitope of influenza A virus is mutated by a substitution of the amino acid sequence or an occurrence of a different sugar chain modification in the influenza virus subtype H5N1, and thus cannot be specifically recognized by the aforementioned second antibody. Such a monoclonal antibody has not yet been known, and it is novel.

Accordingly, in accordance with another aspect of the present invention, there is provided a monoclonal antibody which is not reactive with a virulent strain of influenza A virus but is reactive with all influenza A virus subtypes other than the virulent strain. In particular, the monoclonal antibody is not reactive with the nucleoprotein of the influenza A virus subtype H5N1 but is reactive with the nucleoproteins of all influenza A virus subtypes other than the subtype H5N1.

In the present invention, the third antibody used in a sandwich immunoassay such as an immunochromatographic assay may be any antibody, as long as it is reactive with an antigen with which the first antibody is reactive. The third antibody may be identical to the first antibody, or may be an antibody other than the first antibody. For example, when the antigen possesses plural epitopes that are reactive with the first antibody, an antibody identical to the first antibody can be used as the third antibody. Accordingly, the third antibody may be an antibody reactive with all influenza A virus subtypes. Further, the third antibody may be either a monoclonal antibody or a polyclonal antibody, and is preferably a monoclonal antibody, and particularly, a monoclonal antibody reactive with the nucleoprotein of influenza virus from the viewpoint of specificity.

In the present invention, the fourth antibody used in a sandwich immunoassay such as an immunochromatographic assay may be any antibody, as long as it is reactive with an antigen with which the second antibody is reactive. The fourth antibody may be identical to the second antibody, or may be an antibody other than the second antibody. For example, when the antigen possesses plural epitopes that are reactive with the second antibody, an antibody identical to the second antibody can be used as the fourth antibody. Accordingly, the fourth antibody may be an antibody reactive with all influenza A virus subtypes. Otherwise, the fourth antibody may also be an antibody that is not reactive with a virulent strain of influenza A virus but is reactive with all influenza A virus subtypes other than the virulent strain. Further, the fourth antibody may be either a monoclonal antibody or a polyclonal antibody, and is preferably a monoclonal antibody, and particularly, a monoclonal antibody reactive with the nucleoprotein of influenza virus from the viewpoint of specificity. When the fourth antibody is an antibody reactive with all influenza A virus subtypes, it may be identical to the third antibody.

Referring to influenza A virus subtypes, at present, 16 subtypes are known in terms of H (hemagglutinin) protein, and 9 subtypes are known in terms of N (neuraminidase) protein. Thus, theoretically, 144 (16 x 9) subtypes would be present. In general, however, an antibody reactive with the representative examples of subtypes H1 to H15 is understood to be an "antibody reactive with all influenza A virus subtypes." Accordingly, in the present specification, "all influenza A virus subtypes" mean the representative examples of currently known subtypes H1 to H15, and concretely mean the strains shown in Table 2 later in the present specification. Likewise, "all influenza A virus subtypes other than the virulent strain" mean the representative examples of the subtypes H1 to H15, and concretely mean the strains shown in Table 2 later in the present specification, from which virulent strains are excluded. The term "virulent strain" means a subtype H5N1 and virulent strains of subtypes H5N2 and H7N1. The antibody reactive with the nucleoprotein of influenza A virus is not cross-reactive with the nucleoprotein of influenza B virus or influenza C virus. Similarly, neither the first antibody nor the second antibody of the present invention is cross-reactive with the nucleoprotein of influenza B virus or influenza C virus.

In addition, in the present specification, the expression "a positive response in a reaction with an antibody" means that an antigen-antibody reaction is performed between an antigen and the antibody, whereas the expression "a negative response in a reaction with an antibody" means that no antigen-antibody reaction is performed between an antigen and the antibody.
Moreover, in the present specification, the expression "not reactive with a virulent strain of influenza A virus" concerning antibodies includes a case where an antibody performs no antigen-antibody reaction with any zone of at least one selected from the group consisting of influenza A virus subtype H5N1 and virulent strains of subtypes H5N2 and H7N1, and a case where an antibody only slightly performs the antigen-antibody reaction whilst it performs no antigen-antibody reaction in a certain state of use, for example, when the antibody is immobilized in a membrane carrier.

### EFFECT OF THE INVENTION

According to the present invention, by use of an antibody reactive with all influenza A virus subtypes as a first antibody together with an antibody that is not reactive with a virulent strain of influenza A virus but is reactive with all influenza A virus subtypes other than the virulent strain as a second antibody, it has become possible to detect an antigen that shows a positive response in a reaction with the first antibody and shows a negative response in a reaction with the second antibody according to an immunoassay. This detection method can be used to selectively or specifically detect a virulent strain of influenza virus, and can be applied to a wide range of diagnoses of infectious diseases caused by virulent strains of influenza viruses to humans, birds and the like.

Moreover, by use of the immunochromatographic assay and immunochromatographic assay device of the present invention, it becomes possible to simply and rapidly detect a virulent strain of influenza virus and diagnose infection with the virus in a hospital, a poultry farm, etc., without special equipment or skilled techniques.

### BEST MODE FOR CARRYING OUT THE INVENTION

An assay device that can be used to carry out the immunochromatographic assay of the present invention can be easily practiced based on the structure of a known immunochromatographic test strip. Hereinafter, specific examples thereof will be described with reference to the drawings.

### (1) Embodiment using a single immunochromatographic test strip

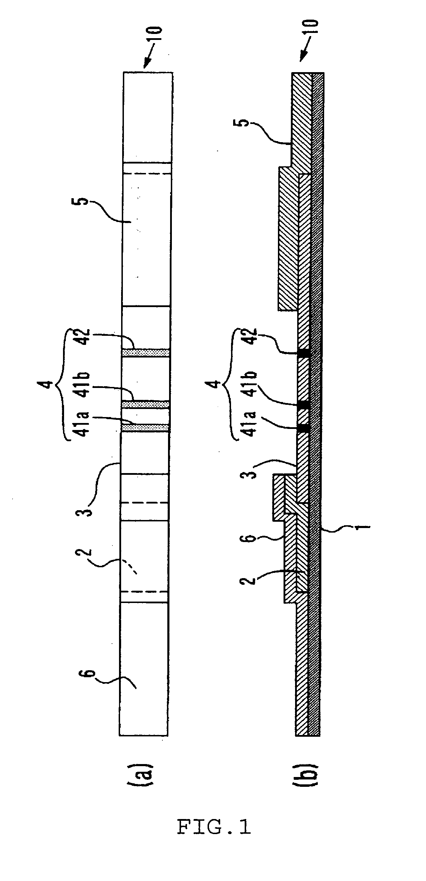

As a specific example of an immunochromatographic test strip, mention may be made of, for example, a test strip 10 as shown in Figure 1. In Figure 1, the numeral 1 indicates an adhesive sheet, 2 indicates an impregnated member, 3 indicates a membrane carrier, 4 indicates a capturing zone, 5 indicates an adsorbing member, and 6 indicates a sample-receiving member.
In the example as shown in the figure, the membrane carrier 3 consists of an elongated strip-shaped nitrocellulose membrane filter having a width of 5 mm and a length of 36 mm. The membrane carrier 3 is attached to the middle of the adhesive sheet 1 having the same width as above. The membrane carrier 3 has a first antibody which is immobilized at a position 7.5 mm downstream from the side of the starting point of chromatographic development, namely, from the end of the membrane carrier on the left side in Figure 1 (which is hereinafter referred to as an "upstream side," and the opposite side, namely, the side of the chromatographic development direction, that is, the right side in Figure 1 is hereinafter referred to as a "downstream side"). Thus, a first capturing zone 41a is formed for capturing a complex of a third antibody and a virus antigen such as the nucleoprotein of influenza A virus. Moreover, a second antibody is immobilized at a position 10.5 mm downstream from the end on the upstream side of the membrane carrier 3, and thus, a second capturing zone 41b is formed for capturing a complex of a fourth antibody (which may be identical to the third antibody in the device of Figure 1, as described later) and a virus antigen such as the nucleoprotein of influenza A virus other than a virulent strain. Furthermore, a so-called control line 42 is provided at a position 15.0 mm downstream from the end on the upstream side of the membrane carrier 3. This control line 42 is provided to confirm that the reaction has been carried out regardless of the presence or absence of an analyte. In general, such a control line 42 can be formed by immobilizing a substance that is capable of immunologically binding specifically to the third and/or fourth antibodies (except for the analyte) in the membrane carrier 3. For example, when mouse antibodies are used as the third and/or fourth antibodies, an antibody against the mouse antibodies can be used as the substance.

In the device of Figure 1, a nitrocellulose membrane filter is used as the membrane carrier 3. However, any type of membrane carrier can be used herein, as long as it is able to chromatographically develop an analyte contained in a test sample and immobilize an antibody that forms the capturing zone 4. Thus, other types of cellulose membranes, nylon membranes, glass fiber membranes, or the like can also be used.
In the device of Figure 1, as the first antibody, a monoclonal antibody reactive with the nucleoproteins of all influenza A virus subtypes is preferably used. As the second antibody, a monoclonal antibody reactive with the nucleoproteins of all influenza A virus subtypes other than a virulent strain is preferably used.

In the present invention, the third antibody is an antibody reactive with an antigen with which the first antibody is reactive, and is preferably an antibody reactive with the nucleoproteins of all influenza A virus subtypes. In many cases, an antibody identical to the first antibody can be used as the third antibody. In addition, the fourth antibody is an antibody reactive with an antigen with which the second antibody is reactive. Examples of the fourth antibody used herein include a monoclonal antibody reactive with the nucleoproteins of all influenza A virus subtypes and a monoclonal antibody reactive with the nucleoproteins of all influenza A virus subtypes other than a virulent strain. That is to say, the fourth antibody is an antibody that may be identical to the first, second or third antibody. In the device of Figure 1, as the third and fourth antibodies which may be identical to or different from each other, antibodies that are reactive with all influenza A virus subtypes are used. In many cases, as the third and fourth antibodies, a monoclonal antibody identical to the first antibody can be used.

The third and fourth antibodies may be mixed with a test sample and a developing solvent to prepare a mixed solution in a suitable vessel that is separate from the immunochromatographic test strip 10. Thereafter, the mixed solution may be injected into the sample-receiving member 6 of the immunochromatographic test strip 10 shown in the figure, so that it is chromatographically developed in the membrane carrier 3. However, the third and fourth antibodies are preferably integrated with the immunochromatographic test strip 10, so that when a mixed solution prepared by mixing a test sample with a developing solvent is injected.into the sample-receiving member 6, these antibodies can be mixed with the mixed solution and chromatographically developed in the membrane carrier 3. Hence, in the device of Figure 1, the third and fourth antibodies have previously been labeled with a suitable labeling agent, and then are impregnated with a member 2 which is communicated with the end of the upstream side of the membrane carrier 3, whereby these antibodies constitute part of the immunochromatographic test strip 10. It is to be noted that the third and/or fourth antibodies that are in a labeled state are often referred to as labeled antibodies in the present specification.
In the example as shown in the figure, a strip-shaped glass fiber nonwoven fabric having a size of 5 mm x 15 mm is used as the impregnated member 2. However, the impregnated member 2 is not limited thereto, but includes, for example, cellulose fabrics (a filter, a nitrocellulose membrane, etc.), porous plastic fabrics such as of polyethylene and polypropylene, and others.

As a labeling agent that labels the third and fourth antibodies, any substance can be used, as long as it is usable herein. Examples of such a labeling agent include a color labeling agent, an enzyme labeling agent, and a radiation labeling agent.
Of these, a color labeling agent is preferably used because observation of a color change in the capturing zone 4 with naked eyes enables rapid and simple determination.
Examples of such a color labeling agent include metallic colloidal particles such as a gold colloidal particle and a platinum colloidal particle, synthetic latex particles such as polystyrene latex dyed with red or blue pigment, and latex particles such as natural rubber latex. Of these, metallic colloidal particles such as a gold colloidal particle are particularly preferable.
The impregnated member 2 can be produced by absorbing a suspension of a labeled antibody into a member such as the aforementioned glass fiber nonwoven fabric, and then drying it.

As shown in Figure 1, the immunochromatographic test strip 10 can be produced by affixing the membrane carrier 3 to the middle of the adhesive sheet 1, laying the downstream side end of the impregnated member 2 on the upstream side end of the membrane carrier 3 so as to communicate them, and affixing the upstream side zone of the impregnated member 2 to the adhesive sheet 1.
Moreover, in the device of Figure 1, the downstream side zone of the sample-receiving member 6 is placed on the upper face of the impregnated member 2, and at the same time, the upstream side zone of the sample-receiving member 6 is affixed to the adhesive sheet 1. Furthermore, the upstream side zone of the absorbing member 5 is placed on the upper face of the downstream side zone of the membrane carrier 3, and at the same time, the downstream side zone of the absorbing member 5 is affixed to the adhesive sheet 1. Thus, the immunochromatographic test strip 10 is constructed.

As the sample-receiving member 6, may be used, for example, sheets or films of porous synthetic resins such as porous polyethylene and porous polypropylene, and cellulose papers or woven or nonwoven fabrics such as a filter and a cotton fabric.
The absorbing member 5 may be made of any material as long as it is able to quickly absorb and retain a liquid. Examples of such a material include cotton fabrics, filters, and porous plastic nonwoven fabrics made from polyethylene, polypropylene, etc. In particular, a filter is optimal.

The immunochromatographic test strip 10 as shown in Figure 1 may be directly used as a dipstick measurement device. Alternatively, the immunochromatographic test strip 10 may be supplied as a dipstick measurement device that is backed or sandwiched with an appropriate plastic sheet(s). Preferably, the immunochromatographic test strip 10 as shown in Figure 1 is provided as a measurement device which houses the test strip in a suitable plastic casing having a test sample-injecting zone and a determining zone opened above the sample-receiving member 6 and the capturing zone 4, respectively. As stated above, in the present invention, the third and fourth antibodies may be mixed with a test sample and a developing solvent in a suitable vessel separate from the immunochromatographic test strip 10 so as to produce a mixed solution, and the mixed solution may be then injected into the sample-receiving zone 6 of the immunochromatographic test strip 10 so that it may be chromatographically developed in the membrane carrier 3. Thus, when the immunochromatographic test strip 10 is housed in a casing, the third and fourth antibodies may be absorbed into the impregnated member 2, and thus may be stored inside the aforementioned casing. Otherwise, the third and fourth antibodies may be contained in a suitable vessel separate from the aforementioned casing, and thus they may be stored outside the aforementioned casing.

Thus, a test sample consisting of a biological sample or the like of a patient suspected of infection with a virulent strain such as influenza A virus subtype H5N1 is mixed with a suitable developing solvent, as necessary, so as to obtain a mixed solution that can be developed chromatographically. Thereafter, the mixed solution is injected into the sample-receiving member 6 of the immunochromatographic test strip 10 as shown in Figure 1, so that it passes through the sample-receiving member 6 and is mixed with a labeled antibody at the impregnated member 2.
In this instance, if a virulent strain of influenza A virus exists in the aforementioned mixed solution, a complex of the virus antigen of the virulent virus strain and the labeled antibody is formed as a result of an antigen-antibody reaction. This complex is developed chromatographically in the membrane carrier 3, and then reaches the capturing zone 4. Thus, the complex is captured by the first antibody immobilized in the first capturing zone 41a as a result of an antigen-antibody reaction to give a positive result. However, the complex is not captured by the second antibody immobilized in the second capturing zone 41b to give a negative result. In contrast, if influenza A viruses other than the virulent strain exist in the mixed solution, a positive result is obtained in both the first capturing zone 41a and the second capturing zone 41b. Accordingly, by piecing together the results of the first and second capturing zones, the infection with a virulent strain and the infection with influenza A virus other than the virulent strain can be differentially determined.

If a color labeling agent such as a gold colloidal particle is used as a labeling agent, whether the result is positive or negative can be immediately determined based on coloring caused by accumulation of the color labeling agent at the first and second capturing zones 41a and 41b.
In the device of Figure 1, since the second capturing zone 41b is located downstream of the first capturing zone 41a, the detection sensitivity of the second capturing zone 41b tends to decrease. Thus, it is preferable that the sensitivity be controlled, for example, by using an antibody immobilized in the second capturing zone 41b in an amount larger than that of an antibody immobilized in the first capturing zone 41a.

The test sample is not particularly limited, but includes, for example, a cloacal swab, a tracheal swab, feces, a nasal aspirate fluid, a nasal swab, a throat swab, blood (which may be whole blood, serum, or blood plasma), saliva, urine, and an organ emulsion. The test sample may be diluted with a suitable diluent such as a developing solvent before it is injected into the membrane carrier.
When whole blood is used as a test sample, it is preferred that a hematocyte-capturing membrane member is disposed in the aforementioned sample-receiving member if a color labeling agent such as a gold colloidal particle is particularly used as a labeling agent for a labeled antibody. Such a hematocyte-capturing membrane member is preferably laminated between the aforementioned impregnated member and the aforementioned sample-receiving member. This inhibits development of erythrocytes in the membrane carrier and thus facilitates the confirmation of accumulation of color labeling agents in the capturing zone of the membrane carrier. As such a hematocyte-capturing membrane member, a carboxymethyl cellulose membrane is used. Specifically, an ion exchange filter CM (trade name) available from Advantec Toyo K.K., an ion exchange cellulose paper available from Whatman Japan K.K., etc. can be used.

### (2) Embodiment using two immunochromatographic test strips

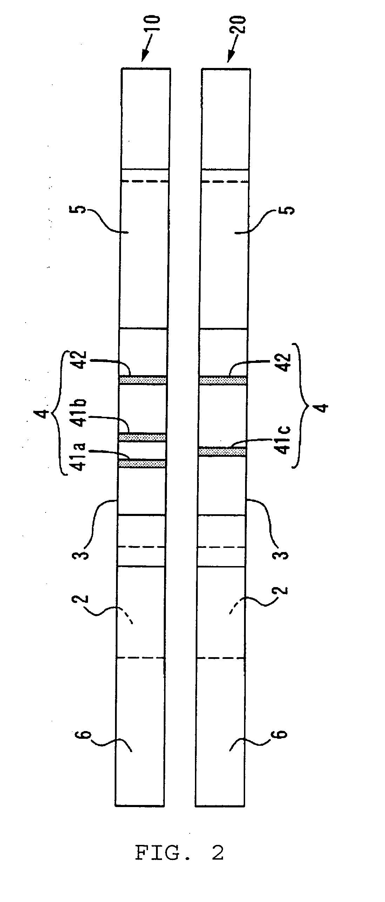

The device of Figure 2 is an immunochromatographic assay device which comprises two immunochromatographic test strips 10 and 20. Each of the immunochromatographic test strips 10 and 20 has the same structure as that of the immunochromatographic test strip 10 shown in Figure 1, except for the structures of a capturing zone 4 and labeled antibodies. The immunochromatographic test strip 10 shown in Figure 2 is the same as Figure 1 in that a first antibody is immobilized at a position 7.5 mm from the end on the upstream side of a membrane carrier 3, so that a first capturing zone 41a is formed to capture a complex of a third antibody and an analyte. However, the immunochromatographic test strip 10 shown in Figure 2 is different from Figure 1 in that an antibody against influenza B virus is immobilized at a position 10.5 mm from the end on the upstream side of the membrane carrier 3, so that a capturing zone 41b for detecting influenza B virus is formed. In addition, labeled antibodies composed of a labeled third antibody and a labeled anti-influenza B virus antibody are absorbed into the impregnated member 2 of the immunochromatographic test strip 10. Herein, as the third antibody, an antibody identical to the first antibody can be used in many cases. As the anti-influenza B virus antibody, a known antibody that has been used for an immunochromatographic assay device can be used.
The immunochromatographic test strip 20 shown in Figure 2 is different from the immunochromatographic test strip 10 shown in Figure 1 in that a second antibody is immobilized at a position 9.0 mm from the end on the upstream side of a membrane carrier 3, so that a second capturing zone 41c is formed to capture a complex of a fourth antibody and an analyte. In addition, a labeled antibody composed of a labeled fourth antibody is absorbed into an impregnated member 2 of the immunochromatographic test strip 20. Herein, as the fourth antibody, an antibody identical to the second antibody is used. However, an antibody identical to the first antibody may also be used as the fourth antibody.

Thus, after a mixed solution containing a test sample is obtained by the same method as described above referring to Figure 1, the mixed solution is injected into a sample-receiving member 6 of each of the immunochromatographic test strips 10 and 20 shown in Figure 2, so that it passes through the sample-receiving member 6, and is mixed with a labeled antibody at the impregnated member 2.
In this instance, if a virulent strain of influenza A virus exists in the aforementioned mixed solution, a complex of the virus antigen of the virulent strain and the third antibody is formed as a result of an antigen-antibody reaction in the immunochromatographic test strip 10. This complex is developed chromatographically in the membrane carrier 3, and then reaches the capturing zone 4. Thus, the complex is captured by the first antibody immobilized in the first capturing zone 41a as a result of an antigen-antibody reaction to give a positive result. However, the complex is not captured by the anti-influenza B virus antibody immobilized in the capturing zone 41b to give a negative result. In the immunochromatographic test strip 20, a complex of the virus antigen of the virulent strain and the fourth antibody is not formed, and thus each of the antigen and the antibody is independently developed chromatographically in the membrane carrier 3, and reaches the second capturing zone 41c. However, they are not captured by the second antibody immobilized therein to give a negative result.

In contrast, if an influenza A virus other than the virulent strain exists in the mixed solution, a complex of the virus antigen and the third antibody is formed in the immunochromatographic test strip 10 as a result of an antigen-antibody reaction. This complex is developed chromatographically in the membrane carrier 3, and reaches the capturing zone 4. Then, the complex is captured by the first antibody immobilized in the first capturing zone 41a as a result of an antigen-antibody reaction to give a positive result. However, the complex is not captured by the anti-influenza B virus antibody immobilized in the capturing zone 41b to give a negative result. In the immunochromatographic test strip 20, a complex of the virus antigen and the fourth antibody is formed. The complex is developed chromatographically.in the membrane carrier 3, and reaches the second capturing zone 41c. Then, the complex is captured by the second antibody immobilized therein as a result of an antigen-antibody reaction to give a positive result.

Moreover, if an influenza B virus exists in the mixed solution, a complex of the virus antigen and an anti-influenza B virus antibody is formed in the immunochromatographic test strip 10 as a result of an antigen-antibody reaction. This complex is developed chromatographically in the membrane carrier 3, and reaches the capturing zone 4. Then, the complex is captured by the anti-influenza B virus antibody immobilized in the capturing zone 41b as a result of an antigen-antibody reaction to give a positive result. However, the complex is not captured by the first antibody immobilized in the first capturing zone 41a to give a negative result. In the immunochromatographic test strip 20, a complex of the virus antigen and the fourth antibody is not formed, and thus each of the antigen and the antibody is independently developed chromatographically in the membrane carrier 3, and reaches the second capturing zone 41c. However, they are not captured by the second antibody immobilized therein to give a negative result.
Accordingly, based on the results obtained in the first capturing zone 41a, the second capturing zone 41c, and the capturing zone 41b, the infection with a virulent strain, the infection with influenza A virus other than the virulent strain, and the infection with influenza B virus can be differentially determined.

The device of Figure 2 only has to comprise at least the two immunochromatographic test strips 10 and 20. The two strips may be housed in a single casing. Otherwise, several pieces of the respective strips may be contained in respective single casings, so that one piece of the strip may be taken out of the respective casings when it is used. In the former case, an integrated product may be formed, which comprises a casing in which the immunochromatographic test strips 10 and 20 are disposed in a manner that the membrane carriers thereof are in parallel with each other whilst the impregnated members thereof are spaced at a certain distance from each other.
As the immunochromatographic test strip 10 as shown in Figure 2, a commercially available immunochromatographic test strip or kit for differential determination of influenza type-A and type-B, such as CAPILIA (registered trademark) FluA+B (manufactured by Tauns Laboratories, Inc.), can be used as it is. Accordingly, only the immunochromatographic test strip 20 as shown in Figure 2 may be supplied as a single item, so that the detection method or assay of the present invention may be carried out using the strip in combination with such a commercially available test strip or kit.

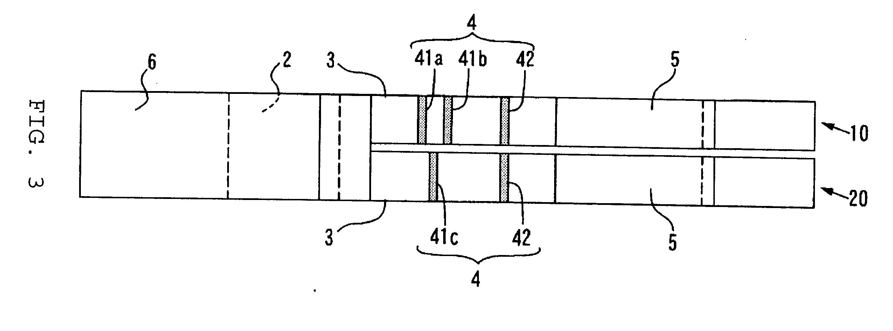

The device of Figure 3 has the same structure as that of the device of Figure 2, except that immunochromatographic test strips 10 and 20 share a single impregnated member 2 and a single sample-receiving member 6. That is, both membrane carriers 3 of the immunochromatographic test strips 10 and 20 are disposed adjacent to and substantially in parallel with each other. At the end on the upstream side, a single wide (width: approximately 12 mm) impregnated member 2 is extended on and communicated with both the membrane carriers 3. In this impregnated member 2, the third and fourth antibodies and an anti-influenza B virus antibody are disposed so as to be developed chromatographically in the same manner as described above. In this embodiment, similarly to the device of Figure 1, it is preferred that both the third and fourth antibodies are antibodies reactive with all influenza A virus subtypes, and it is convenient that both the third and fourth antibodies are identical to the first antibody.

Thus, after a mixed solution containing a test sample is obtained by the same method as described above referring to Figure 2, the mixed solution is injected into the sample-receiving member 6 shared by the immunochromatographic test strips 10 and 20 shown in Figure 3, so that it passes through the sample-receiving member 6, and is mixed with a labeled antibody at the impregnated member 2.
In this instance, if a virulent strain of influenza A virus exists in the aforementioned mixed solution, the immunochromatographic test strip 10 gives a positive result in the first capturing zone 41a and a negative result in the capturing zone 41b similarly to Figure 2. In the immunochromatographic test strip 20, although a complex of the virus antigen of the virulent strain and the labeled antibody is formed, it is not captured by the second antibody immobilized in the second capturing zone 41c to give a negative result. If influenza A viruses other than the virulent strain exist in the mixed solution, the immunochromatographic test strip 10 gives a positive result in the first capturing zone 41 and a negative result in the capturing zone 41b similarly to Figure 2. In the immunochromatographic test strip 20, a complex of the virus antigen and the labeled antibody is formed and developed chromatographically in the membrane carrier 3, and reaches the second capturing zone 41c. Then, the complex is captured by the second antibody immobilized therein to give a positive result.

Moreover, if an influenza B virus exists in the mixed solution, the immunochromatographic test strip 10 gives a positive result in the capturing zone 41b and a negative result in the first capturing zone 41a similarly to Figure 2. The immunochromatographic test strip 20 gives a negative result in the second capturing zone 41c similarly to Figure 2.
Accordingly, based on the results obtained in the first capturing zone 41a, the second capturing zone 41c, and the capturing zone 41b, the infection with a virulent strain, the infection with influenza A virus other than the virulent strain, and the infection with influenza B virus can be differentially determined.
The device of Figure 3 may also be housed in a suitable casing.

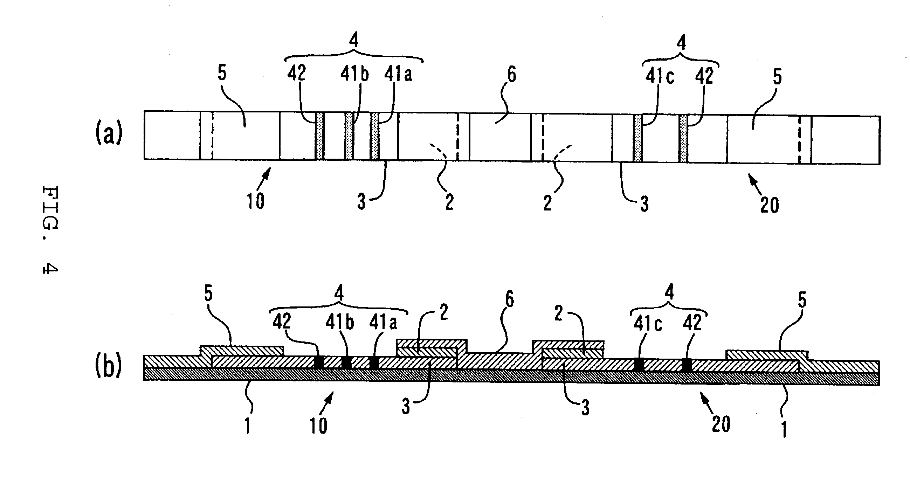

The device of Figure 4 has the same structure as that of Figure 2, except that immunochromatographic test strips 10 and 20 share a single adhesive tape 1 and a single sample-receiving member 6. That is, both membrane carriers 3 of the immunochromatographic test strips 10 and 20 are substantially linearly disposed in the direction opposite to each other. Specifically, the immunochromatographic test strips 10 and 20 are disposed with the ends on the upstream side of the impregnated members 2 of the two test strips facing each other at a suitable distance, namely, the two strips are positioned oppositely to each other on a straight line. A single sample-receiving member 6 is extended on and communicated with the two impregnated members 2. Alternatively, the two immunochromatographic test strips 10 and 20 may be disposed radially such that the membrane carriers of the two test strips extend at an angle to each other.
Thus, after a mixed solution containing a test sample is obtained by the same method as described above referring to Figure 2, the mixed solution is injected into the sample-receiving member 6 shared by the immunochromatographic test strips 10 and 20 shown in Figure 4, so that it passes through the sample-receiving member 6, and is mixed with a labeled antibody at the impregnated members 2 of the immunochromatographic test strips 10 and 20. Then, based on the results obtained in the first capturing zone 41a, the second capturing zone 41c, and the capturing zone 41b, the infection with a virulent strain, the infection with influenza A virus other than the virulent strain, and the infection with influenza B virus can be differentially determined similarly to Figure 2.

The device of Figure 4 may also be housed in a suitable casing.
Referring to a modification of the device of Figure 4, not only the adhesive sheet 1 and the sample-receiving member 6 but also a single impregnated member may be shared by the immunochromatographic test strips 10 and 20. In this case, the third and fourth antibodies may be constituted in accordance with the embodiment of Figure 3 to practice the present invention.
In the device of Figure 4, the aforementioned mixed solution containing the test sample is immersed into the test strips 10 and 20 uniformly. Thus, unlike the device of Figure 1, the device of Figure 4 is convenient in that it is unnecessary to control the sensitivity of the first and second capturing zones 41a and 41b.

### (3) Embodiment using the second antibody as the labeled antibody

The device of Figure 2 can be modified to a device for carrying out an embodiment of the present invention using the second antibody as the labeled antibody. Such a device can be produced by immobilizing a first antibody, instead of a second antibody, in the second capturing zone 41c of the immunochromatographic test strip 20 of the device of Figure 2, and using a second antibody, instead of a fourth antibody, as a labeled antibody to be absorbed into the impregnated member 2 of the aforementioned strip 20. In this device, the structure of the immunochromatographic test strip 10 is completely identical to that of Figure 2.
Thus, after a mixed solution containing a test sample is obtained by the same method as described above referring to Figure 2, the mixed solution is injected into the sample-receiving member 6 of each of the immunochromatographic test strips 10 and 20 of the modified device of Figure 2, so that it passes through the sample-receiving member 6, and is mixed with a labeled antibody at the impregnated members 2.

In this instance, if a virulent strain of influenza A virus exists in the aforementioned mixed solution, the immunochromatographic test strip 10 gives a positive result in the first capturing zone 41a and a negative result in the capturing zone 41b similarly to Figure 2. In the immunochromatographic test strip 20, a complex of the virus antigen of the virulent strain and the labeled antibody is not formed, and thus the antigen and the antibody are independently developed chromatographically in the membrane carrier 3, and reach the second capturing zone 41c. The virus antigen of the virulent strain is captured by the first antibody immobilized in the second capturing zone 41c. However, since the labeled antibody does not bind thereto, it gives a negative result.
If influenza A viruses other than the virulent strain exist in the mixed solution, the immunochromatographic test strip 10 gives a positive result in the first capturing zone 41 and a negative result in the capturing zone 41b similarly to Figure 2. In the immunochromatographic test strip 20, a complex of the virus antigen and the labeled antibody is formed, and is developed chromatographically in the membrane carrier 3 and reaches the capturing zone 41. Then, the complex is captured by the first antibody in the second capturing zone 41c to give a positive result.

Moreover, if an influenza B virus exists in the mixed solution, the immunochromatographic test strip 10 gives a positive result in the capturing zone 41b and a negative result in the first capturing zone 41a similarly to Figure 2. The immunochromatographic test strip 20 gives a negative result in the second capturing zone 41c.
Accordingly, based on the results obtained in the first capturing zone 41a, the second capturing zone 41c, and the capturing zone 41b, the infection with a virulent strain, the infection with influenza A virus other than the virulent strain, and the infection with influenza B virus can be differentially determined.

In accordance with the same modification as described above, the device of Figure 4 can also be modified to a device for carrying out the embodiment of the present invention using the second antibody as the labeled antibody. That is, such a modification can be made to the device of Figure 4 by immobilizing the first antibody, instead of the second antibody, in the second capturing zone 41c of the immunochromatographic test strip 20, and using the second antibody, instead of the fourth antibody, as a labeled antibody to be absorbed into the impregnated member 2 of the aforementioned strip 20. Meanwhile, the structure of the immunochromatographic test strip 10 is completely identical to that of Figure 4.

Thus, after a mixed solution containing a test sample is obtained by the same method as described above referring to Figure 2, the mixed solution is injected into a sample-receiving member 6 shared by the immunochromatographic test strips 10 and 20 of the modified device of Figure 4, so that it passes through the sample-receiving member 6, and is mixed with a labeled antibody at the impregnated members 2 of the test strips 10 and 20. Similarly to the aforementioned modified device of Figure 2, based on the results obtained in the first capturing zone 41a, the second capturing zone 41c, and the capturing zone 41b, the infection with a virulent strain, the infection with influenza A virus other than the virulent strain, and the infection with influenza B virus can be differentially determined.

### EXAMPLES

The present invention will be more specifically described in the following examples. However, these examples are not intended to limit the scope of the present invention.

### Example 1 (Cloning of recombinant nucleoprotein (NP) genes of influenza A and B viruses)

Influenza A virus, A/Puerto Rico/8/34 (H1N1) and influenza B virus, B/Lee/40 were inoculated in embryonated chicken eggs, and were then cultured. Several days later, a chorioallantoic fluid was collected to obtain viruses. An RNA extraction reagent (TRIzol Reagent, Invitrogen) was added to such viruses, and each virus RNA was extracted. A reverse transcription reaction was performed on the extracted RNA, so as to produce NP gene cDNA. Uni 12 Primer: 5'-AGCAAAAGCAGG-3' (SEQ ID NO: 1) was used as a primer in the reverse transcription reaction.

The obtained NP gene cDNAs of A/Puerto Rico/8/34 and B/Lee/40 were subjected to a polymerase chain reaction (PCR) method, so as to amplify the NP genes. In the case of A/Puerto Rico/8/34, the following primers were used: a sense primer: 5'-GGAATTCCATATGGCGTCCCAAGGCACC-3' (SEQ ID NO: 2); and an antisense primer: 5'-CCGCTCGAGTTAATTGTCGTACTCCTCTGC-3' (SEQ ID NO: 3). In the case of B/Lee/40, the following primers were used: a sense primer: 5'-CGGGATCCATATGTCCAACATGGATATTG-3' (SEQ ID NO: 4); and an antisense primer: 5'-CCGCTCGAGTTAATAATCGAGGTCATCATA-3' (SEQ ID NO: 5). For incorporation into a vector pET15b, Nde I and Xho I sites were added to the primers as the upper primer and the lower primer, respectively.
A portion of the obtained PCR product was subjected to DNA electrophoresis to confirm its amplification, and each amplified product was then cleaved with restriction enzymes Nde I and Xba I. The cleaved product was then inserted into the vector pET15b cleaved with the same restriction enzymes.

### Example 2 (Expression of recombinant nucleoproteins (NP proteins) and purification thereof)

*E. coli* BL21 CodonPlus was transformed with the vector pET15b into which the gene of interest had been inserted in Example 1. It was then cultured on an ampicillin selective medium, so as to obtain a colony. It was cultured overnight in L-Broth containing ampicillin (100 µg/ml) and chloramphenicol (25 µg/ml), and the culture was then added to L-broth containing ampicillin (100 µg/ml). The mixture was cultured at 37°C for 3 hours, and 1 M IPTG was then added thereto to a final concentration of 1 mM, so as to induce expression of the recombinant NP protein. Expression of the protein by IPTG was confirmed by SDS-PAGE.
A cell mass recovered by centrifugation was suspended in a solubilizing buffer. The suspension was further subjected to PMSF and a lysozyme treatment, so as to completely dissolve the cell mass. A recombinant NP protein was obtained from the centrifuged supernatant of the obtained solution, and it was passed through a column containing a Ni-NTA resin, so as to purify it. The purified protein was dialyzed against PBS, so as to obtain a recombinant NP protein of interest.

### Example 3 (Production of monoclonal antibody)

Using the influenza A virus recombinant NP protein (Flu A NP) and influenza B virus recombinant NP protein (Flu B NP) obtained in Example 2 as antigens, monoclonal antibodies against Flu A NP and Flu B NP (an anti-Flu A NP antibody and an anti-Flu B NP antibody) were produced. Such monoclonal antibodies were produced according to an ordinary method. 100 µg of each recombinant NP protein was mixed with the same amount of Adjuvant Complete Freund (Difco). Thereafter, a mouse (BALB/c, 5-week-old, Japan SLC, Inc.) was immunized with the mixture 3 times, and the splenic cells thereof were then used in cell fusion.
For such cell fusion, Sp2/0-Ag14 cells (Shulman et al., 1978) that were mouse myeloma cells were used. For the culture of the cells, there was used a culture medium which was Dulbecco's Modified Eagle Medium (Gibco) (DMEM) containing 0.3 mg/ml L-glutamine, 100 units/ml penicillin G potassium, 100 µg/ml streptomycin sulfate and 40 µg/ml Gentacin, to which fetal bovine serum (JRH) was further added to a concentration of 10%.

Cell fusion was carried out by mixing the splenic cells of the immunized mouse with Sp2/0-Ag14 cells, and then adding a polyethylene glycol solution (Sigma) thereto. The fused cells were cultured in HAT-DMEM (serum-added DMEM containing 0.1 mM sodium hypoxanthine, 0.4 µM aminopterin and 0.016 mM thymidine (Gibco)). An anti-Flu A NP antibody was screened by confirming that the antibody was produced in the culture supernatant in accordance with the Enzyme-Linked ImmunoSorbent Assay (ELISA) using a plate on which inactivated influenza virus, type A (H1N1) had been immobilized. In addition, an anti-Flu B NP antibody was screened by confirming that the antibody was produced in the culture supernatant in accordance with the same method as above using a plate on which inactivated influenza B virus had been immobilized.

Cells showing that production of antibody was positive were cultured in HT-DMEM (serum-added DMEM containing 0.1 mM sodium hypoxanthine and 0.016 mM thymidine (Gibco)), and further continuously cultured in the serum-added DMEM. Cells having high ability to produce antibodies were selected by the ELISA method, and then cloned. The cloned cells were inoculated into the abdominal cavity of a mouse (BALB/c, Retire, Japan SLC, Inc.) into which 2,6,10,14-tetramethylpentadecane (Sigma) had been inoculated, and the ascites thereof was then collected. Thereafter, IgG purification was carried out by an ordinary method using a protein G adsorbent, so as to obtain monoclonal antibodies. The isotype of the produced monoclonal antibodies was identified by ELISA using Mouse Monoclonal Antibody Isotyping Reagents (Sigma).

Finally, 3 clones of monoclonal antibody-producing cells against Flu A NP, namely, F/169, B/347 and F/211 were obtained. Among them, the antibody-producing cell F/211 was finally found to be a clone that produces an antibody that is not reactive with an influenza A virus subtype H5N1 but is reactive with all influenza A virus subtypes other than the subtype H5N1 (the second antibody of the present invention), as evidenced in the following examples. Four clones of cells producing monoclonal antibodies against Flu B NP were obtained. Of these, only F/171 was used in the following examples.

### Example 4 (Production of immunochromatographic assay device)

### (1) Preparation of anti-Flu A NP antibody, anti-Flu B NP antibody, and anti-Flu A NP antibody that is not reactive with subtype H5N1

Each of the anti-Flu A NP antibody-producing cells F/169 and B/347 obtained in Example 1 was inoculated into the abdominal cavity of a mouse, so as to obtain ascites containing an anti-Flu A NP antibody. In addition, anti-Flu B NP antibody-producing cells F/171 were inoculated into the abdominal cavity of a mouse, so as to obtain ascites containing an anti-Flu B NP antibody. Moreover, anti-Flu A NP antibody-producing cells F/211 were inoculated into the abdominal cavity of a mouse, so as to obtain ascites containing an anti-Flu A NP antibody. Furthermore, IgG purification was carried out according to an ordinary method using a protein G adsorbent, so as to obtain each antibody.

### (2) Preparation of gold colloidal particle solution

1 ml of a 1% (v/w) chloroauric acid aqueous solution was added to 99 ml of extra pure water boiled by heating. One minute later, 1.5 ml of a 1% (v/w) sodium citrate aqueous solution was further added thereto, and the mixture was then heated and boiled for 5 minutes. Thereafter, the solution was left at a room temperature, so that it was cooled. Subsequently, a 200 mM potassium carbonate aqueous solution was added to the solution, so that the solution was adjusted to pH 9.0. Thereafter, extra pure water was added thereto to a total amount of 100 ml, thereby obtaining a gold colloidal particle solution.

### (3) Preparation of gold colloidal particle-labeled antibody solution

The antibody-producing cell B/347-derived anti-Flu A NP antibody (hereinafter referred to as an anti-Flu A NP antibody B/347), the antibody-producing cell F/171-derived anti-Flu B NP antibody (hereinafter referred to as an anti-Flu B NP antibody F/171), and the antibody-producing cell F/211-derived anti-Flu A NP antibody (hereinafter referred to as an anti-Flu A NP antibody F/211), which were obtained in (1) above, were labeled with gold colloidal particles by the following procedures.
3 µg (equivalent to weight of protein) of the anti-Flu A NP antibody B/347 was mixed with 1 ml of the gold colloidal particle solution described in (2) above, and the mixture was then left at rest at a room temperature for 2 minutes, so that all the antibodies were allowed to bind to the surfaces of the gold colloidal particles. Thereafter, a 10% BSA aqueous solution was added to the gold colloidal solution to a final concentration of 1%, and the residual surfaces of the gold colloidal particles were all blocked with the BSA, so as to prepare a gold colloidal particle-labeled anti-Flu A NP antibody B/347 solution. This solution was centrifuged (5600 x G, 30 minutes) to precipitate a gold colloidal particle-labeled antibody. A supernatant solution was eliminated, so as to obtain the gold colloidal particle-labeled antibody. This gold colloidal particle-labeled antibody was suspended in a 50 mM Tris-HCl buffer (pH 7.4) containing 10% saccharose, 1% BSA, and 0.5% Triton-X100, so as to prepare a gold colloid-labeled anti-Flu A NP antibody B/347 solution. In addition, 1 µg (equivalent to weight of protein) of the anti-Flu B NP antibody F/171 was allowed to bind to the surfaces of the gold colloidal particles by the aforementioned method, so as to prepare a gold colloidal particle-labeled anti-Flu B NP antibody F/171 solution. Moreover, 1 µg (equivalent to weight of protein) of the anti-Flu A NP antibody F/211 was allowed to bind to the surfaces of the gold colloidal particles by the aforementioned method, so as to prepare a gold colloidal particle-labeled anti-Flu A NP antibody F/211 solution.

### (4) Production of immunochromatographic assay device

The immunochromatographic assay device as shown in Figure 4 was produced by the following procedures.

### (4-1) Formation of the first capturing zone of the membrane carrier

An elongated strip-shaped nitrocellulose membrane with a size of 5 mm width and 36 mm length was prepared as a membrane carrier 3.
0.5 µl of a solution containing a 1.0 mg/ml anti-Flu A NP antibody F/169 was applied in a linear form to a position 5.0 mm from the end on the upstream side of the membrane carrier 3. It was then dried at a room temperature, so as to form a capturing zone 41a (the first capturing zone) for capturing a complex of a virus antigen of influenza A virus and a labeled antibody. Moreover, 0.5 µl of a solution containing a 1.0 mg/ml anti-Flu B NP antibody F/171 was applied in a linear form to a position 8.0 mm from the end on the upstream side of the membrane carrier 3. It was then dried at a room temperature, so as to form a capturing zone 41b for capturing a complex of a virus antigen of influenza B virus and a labeled antibody.

### (4-2) Formation of the second capturing zone of the membrane carrier

Another membrane carrier 3 identical to the membrane carrier used in (4-1) above was prepared.
0.5 µl of a solution containing a 1.0 mg/ml anti-Flu A NP antibody F/169 was applied in a linear form to a position 5.0 mm from the end on the upstream side of the membrane carrier 3. It was then dried at a room temperature, so as to form a capturing zone 41c (the second capturing zone) for capturing a complex of an influenza virus antigen and a labeled antibody.

### (4-3) Production of the impregnated member impregnated with gold colloidal particle-labeled antibody

A strip-shaped glass fiber nonwoven fabric with a size of 5 mm x 15 mm was impregnated with 37.5 µl of a mixed solution of the gold colloidal particle-labeled anti-Flu A NP antibody B/347 solution and the gold colloidal particle-labeled anti-Flu B NP antibody F/171 solution obtained in (3) above, and was then dried at a room temperature, so as to prepare a labeled antibody-impregnated member. In addition, another strip-shaped glass fiber nonwoven fabric with a size of 5 mm x 15 mm was impregnated with 37.5 µl of a gold colloidal particle-labeled anti-Flu A NP antibody F/211 solution, and was then dried at a room temperature, so as to prepare a labeled antibody-impregnated member: The former labeled antibody-impregnated member was used as the impregnated member 3 of the test strip 10, and the latter labeled antibody-impregnated member was used as the impregnated member 3 of the test strip 20. The membrane carrier obtained in (4-1) above was used as the membrane carrier 3 of the test strip 10, and the membrane carrier obtained in (4-2) above was used as the membrane carrier 3 of the test strip 20. Further, a cotton fabric was used as the sample-receiving member 6, and a filter was used as the absorbing member 5 for each test strip, thereby producing an immunochromatographic assay device having the same configuration as in Figure 4.

### Example 5 (Test for reactivity with influenza virus H5N1 strain (Yamaguchi strain))

The immunochromatographic assay device produced in Example 4 was used to carry out a test for reactivity with the influenza virus H5N1 strain (A/chichen/Yamaguchi/7/04, Yamaguchi strain). Influenza virus H3N2 (A/Aichi/2/68, Aichi strain) was used as a control. There was used a test sample which had been prepared by allowing each of the aforementioned influenza virus strains to grow in an embryonated chicken egg to obtain a chorioallantoic fluid and then diluting the chorioallantoic fluid with an analyte diluent. Using a micropipette, 150 µl of the test sample was added dropwise to the sample-receiving member 6 of the test strip obtained in Example 4, so that it was developed chromatographically therein. It was left at a room temperature for 15 minutes. Thereafter, the amount of the complex of the virus antigen and the gold colloidal particle-labeled antibody captured by each of the first capturing zone 41a and the second capturing zone 41c was observed by naked eyes. The amount of the complex captured was determined by classifying the level of red-violet color, which increases or decreases depending on the captured amount, into the following 5 stages with naked eyes: - (no coloration); ± (slight coloration); + (clear coloration); ++ (clearer coloration); and +++ (significant coloration). It is to be noted that the HA value of the chorioallantoic fluid of the aforementioned Yamaguchi strain was 1024, and that the HA value of the chorioallantoic fluid of the Aichi strain was 2048. The results are shown in Table 1.

**Table 1: Test for reactivity with influenza A virus subtype H5N1**

| Dilution magnification | The second capturing zone | | The first capturing zone | |
|---|---|---|---|---|
| | Yamaguchi strain (HSN1) | Aichi strain (H3N2) | Yamaguchi strain (H5N1) | Aichi strain (H3N2) |
| 10⁻¹ | -∼± | +++ | +++ | +++ |
| 10⁻² | - | ++ | ++ | ++ |
| 10⁻³ | - | + | + | + |
| 10⁻⁴ | - | ± | ± | ± |
| 10⁻⁵ | - | - | - | - |

From the results shown in Table 1, it was found that almost no reactivity with the Yamaguchi strain (H5N1) was confirmed in the second capturing zone 41c, when compared with the reactivity with the Aichi strain (H3N2) used as a positive control. These results suggested that the anti-Flu A NP antibody F/211 used as a labeled antibody for the test strip 20 is not reactive with the Yamaguchi strain (H5N1), and thus it does not specifically react with the influenza A virus subtype H5N1. In contrast, the anti-Flu A NP antibody F/211 had the same level of reactivity with both the Aichi strain (H3N2) and the Yamaguchi strain (H5N1) at the first capturing zone 41a.

### Example 6 (Test for reactivity with various subtypes of influenza A virus)

The immunochromatographic assay device produced in Example 4 was used to carry out a test for reactivity with 15 subtypes of influenza A virus. There was used a test sample which had been prepared by allowing each subtype of influenza A virus to grow in an embryonated chicken egg to obtain a chorioallantoic fluid and then diluting the chorioallantoic fluid with an analyte diluent. Using a micropipette, 150 µl of the test sample was added dropwise to the sample-receiving member 6 of the test strip obtained in Example 4, so that it was developed chromatographically therein. It was left at a room temperature for 15 minutes. Thereafter, the amount of the complex of the virus antigen and the gold colloidal particle-labeled antibody captured by each of the first capturing zone 41a and the second capturing zone 41c was observed by naked eyes. The amount of the complex captured was determined by classifying the level of red-violet color, which increases or decreases depending on the captured amount, into the following 5 stages with naked eyes: - (no coloration); ± (slight coloration); + (clear coloration); ++ (clearer coloration); and +++ (significant coloration).

The following influenza A virus subtypes were subjected to the test: A/Puerto Rico/8/34(H1N1), A/Hokkaido/11/02(H1N1), A/Singapore/1/57(H2N2), A/Aichi/2/68(H3N2), A/Hokkaido/1/03(H3N2), A/duck/Czech/56(H4N6), A/duck/Pennsylvania/1028/83(H5N2), A/Hong Kong/156/97(H5N1), A/Hong Kong/483/97(H5N1), A/turkey/Massachusetts/3740/65(H6N2), A/seal/Massachusetts/1/80(H7N7), A/turkey/Ontario/67(H8N4), A/turkey/Wisconsin/66(H9N2), A/chicken/Germany/N/49(H10N7), A/duck/England/56(H11N6), A/duck/Alberta/60/76(H12N5), A/gull/Maryland/704/77(H13N6), A/mallard/Astrakhan/263/82(H14N5), A/duck/Australia/341/83(H15N8). The results are shown in Table 2.

As is clear from Table 2, a significant coloration was observed at the first capturing zone 41a of the test strip 10 with regard to all the virus strains subjected to the test, but no coloration was observed at the capturing zone 41b thereof with regard to all the virus strains. On the other hand, at the second capturing zone 41c of the test strip 20, no coloration was observed with regard to only the H5N1 strains, namely, A/Hong kong/156/97 (H5N1) and A/Hong Kong/483/97 (H5N1) among all the influenza A viruses subjected to the test, but a significant coloration was observed with regard to the other virus strains subjected to the test including a subtype H5N2, A/duck/Pennsylvania/1028/83 (H5N2). Accordingly, it was found that the immunochromatographic assay device described in Example 4 exhibits reactivity with all the influenza A viruses at the first capturing zone 41a but fails to exhibit reactivity with only the influenza virus subtype H5N1 at the second capturing zone 41c. These results demonstrate that the anti-Flu A NP antibody F/211 used as a labeled antibody of the test strip 20 does not react with the influenza A virus subtype H5N1 but reacts with all influenza A virus subtypes other than the subtype H5N1.

Accordingly, when a sample collected from a patient suspected of influenza infection is subjected to the immunochromatographic assay device described in Example 4, if coloration is observed at the first capturing zone 41a and the second capturing zone 41c, it will be confirmed that the patient is infected with the influenza virus H1N1 or H3N2, and thus it will be suspected that the patient is infected with a usual influenza virus. In addition, if coloration is observed at the first capturing zone 41a but is not observed at the second capturing zone 41c, it will be suspected that the patient is infected with the influenza virus H5N1. Accordingly, using this device, it becomes possible to give appropriate treatments to patients depending on the type of an infectious virus and endeavor to prevent expansion of infection. If coloration is observed at the type-B determination zone of the capturing zone 41b, it will be determined that the patient is suspected of being infected with influenza B virus. If no coloration is observed in all determination zones, it will be determined that the patient is negative with regard to influenza infection.

Thus, according to the present invention, infection with the influenza virus H5N1 can be detected by the combined use of the first antibody reactive with all influenza A virus subtypes and the second antibody that is not reactive with the influenza A virus subtype H5N1 but is reactive with all influenza A virus subtypes other than the subtype H5N1.

**Table 2: Test for reactivity with various subtypes of influenza A virus**

| Subtype | Strain name | HA value | Reactivity | |
|---|---|---|---|---|
| | | | The first capturing zone | The second capturing zone |
| H1 | A/Puerto Rico/8/34 (H1N1) | 256 | +++ | +++ |
| H1 | A/Hokkaido/11/02 (H1N1) | 64 | ++ | ++ |
| H2 | A/Singapore/1/57 (H2N2) | 256 | +++ | +++ |
| H3 | A/Aichi/2/68 (H3N2) | 2048 | +++ | +++ |
| H3 | A/Hokkaido/1/03 (H3N2) | 4 | +++ | +++ |
| H4 | A/duck/Czeck/56 (H4N6) | 512 | +++ | +++ |
| H5 | A/duck/Pennsylvania/1028/83 (H5N2) | 256 | +++ | +++ |
| H5 | A/HongKong/156/97 (H5N1) | 512 | +++ | - |
| H5 | A/HongKong/483/97 (H5N1) | 512 | +++ | - |
| H6 | A/turkey/Massachusetts/3740/65 (H6N2) | 256 | +++ | +++ |
| H7 | A/Seal/Massachusetts/1/80 (H7N7) | 2048 | +++ | +++ |
| H8 | A/turkey/Ontario/67 (H8N4) | 256 | ++ | ++ |
| H9 | A/turkey/Wisconsin/66 (H9N2) | 512 | +++ | +++ |
| H10 | A/chicken/Germany/N/49 (H10N7) | 2048 | +++ | +++ |
| H11 | A/duck/England/1/56 (H11N6) | 512 | ++ | ++ |
| H12 | A/duck/Alberta/60/76 (H12N5) | 512 | +++ | +++ |
| H13 | A/gull/Maryland/704/77 (H13N6) | 512 | +++ | +++ |
| H14 | A/mallard/Astrakhan/263/82 (H14N5) | 1024 | +++ | +++ |
| H15 | A/duck/Australia/341/83 (H15N8) | 128 | +++ | +++ |

### Example 7 (Test for reactivity with various subtypes of influenza A virus in various dilution magnifications)

The immunochromatographic assay device produced in Example 4 was used to carry out a test for reactivity with 8 influenza A viruses. There was used a test sample which had been prepared by allowing each influenza A virus to grow in an embryonated chicken egg to obtain a chorioallantoic fluid and then diluting 10, 100, 1000 and 10000 times the chorioallantoic fluid with an analyte diluent. Using a micropipette, 150 µl of the test sample was added dropwise to the sample-receiving member 6 of the test strip obtained in Example 4, so that it was developed chromatographically therein. It was left at a room temperature for 15 minutes. Thereafter, the amount of the complex of the virus antigen and the gold colloidal particle-labeled antibody captured by each of the first capturing zone 41a and the second capturing zone 41c was observed by naked eyes. The amount of the complex captured was determined by classifying the level of red-violet color, which increases or decreases depending on the captured amount, into the following 6 stages with naked eyes: - (no coloration); ± (slight coloration); + (clear coloration); ++ (clearer coloration); +++ (significant coloration); and ++++ (more significant coloration).

The following influenza A viruses were subjected to the test: A/Vietnam/1194/04(H5N1), A/Hong Kong/483/97(H5N1), A/Chicken/Suphanburi/1/04(H5N1), A/Whooperswan/Mongolia/3/05(H5N1), A/Chicken/Ibaraki/1/05(H5N2), A/Swan/Hokkaido/51/96(H5N3), A/Chicken/Italy/99(H7N1), and A/Chicken/Netherlands/03(H7N7). These influenza A viruses, except for A/Swan/Hokkaido/51/96 (H5N3) and A/Chicken/Netherlands/03 (H7N7), are known as virulent strains. The results are shown in Tables 3-1 to 3-4 together with the HA value of the chorioallantoic fluid of each subtype.

**Table 3-1: Test for reactivity with influenza A viruses**

| Dilution magnification | A/Vietnam/1194/04 (H5N1) HA value = 512 | | A/Hong Kong/483/97 (H5N1) HA value = 512 | |
|---|---|---|---|---|
| | The first capturing zone | The second capturing zone | The first capturing zone | The second capturing zone |
| 10⁻¹ | ++++ | -∼± | ++++ | - |
| 10⁻² | +++ | - | +++ | - |
| 10⁻³ | + | - | ++ | - |
| 10⁻⁴ | ± | - | + | - |

**Table 3-2: Test for reactivity with influenza A viruses**

| Dilution magnification | A/Chicken/Suphanburi/1/04 (H5N1) HA value = 512 | | A/Whooperswan/Mongolia/3/05 (H5N1) HA value = 16 | |
|---|---|---|---|---|
| | The first capturing zone | The second capturing zone | The first capturing zone | The second capturing zone |
| 10⁻¹ | ++++ | + | ++++ | - |
| 10⁻² | ++++ | - | +++ | - |
| 10⁻³ | +++ | - | + | - |
| 10⁻⁴ | + | - | ± | - |

**Table 3-3: Test for reactivity with influenza A viruses**

| Dilution magnification | A/Chicken/Ibaraki/1/05 (H5N2) HA value = 512 | | A/Swan/Hokkaido/51/96 (H5N3) HA value = 256 | |
|---|---|---|---|---|
| | The first capturing zone | The second capturing zone | The first capturing zone | The second capturing zone |
| 10⁻¹ | ++++ | - | +++ | +++ |
| 10⁻² | +++ | - | ++ | ++ |
| 10⁻³ | ++ | - | + | ± |
| 10⁻⁴ | ± | - | ± | - |

**Table 3-4: Test for reactivity with influenza A viruses**

| Dilution magnification | A/Chicken/Italy/99 (H7N1) HA value =512 | | A/Chicken/Netherlands/03 (H7N7) HA value =512 | |
|---|---|---|---|---|
| | The first capturing zone | The second capturing zone | The first capturing zone | The second capturing zone |
| 10⁻¹ | ++++ | ++ | ++++ | ++++ |
| 10⁻² | ++++ | + | +++ | +++ |
| 10⁻³ | ++ | - | ++ | + |
| 10⁻⁴ | - | - | ± | ± |

As is clear from Tables 3-1 to 3-4, coloration was observed at the first capturing zone 41a of the test strip 10 with regard to all the virus strains subjected to the test at a dilution magnification of 1000 times or less. On the other hand, at the second capturing zone 41c of the test strip 20, no coloration was observed with regard to all the H5N1 subtypes and A/Chicken/Ibaraki/1/05 (H5N2) which is a virulent strain of the subtype H5N2, and no coloration was observed with regard to A/Chicken/Italy/99 (H7N1) at a dilution magnification of more than 1000 times, whilst a significant coloration was observed with regard to all the other influenza A virus strains subjected to the test at a dilution magnification of 1000 times or less. Thus, these results demonstrate that the immunochromatographic assay device described in Example 4 is effective for detection of the virulent strains of influenza A virus, preferably for detection of the subtype H5N1 and the virulent strains of subtype H5N2, and particularly preferably for detection of the subtype H5N1.

### INDUSTRIAL APPLICABILITY

The present invention provides a method for detecting a virulent strain of influenza A virus, which uses a first antibody that is reactive with all influenza A virus subtypes and a second antibody that is not reactive with a virulent strain of influenza A virus but is reactive with all influenza A virus subtypes other than the virulent strain, so as to detect an antigen that shows a positive response in a reaction with the first antibody and shows a negative response in a reaction with the second antibody according to an immunoassay. In particular, the present invention provides a sandwich immunoassay, and an immunochromatographic assay and an immunochromatographic assay device. According to the present invention, a virus belonging to a virulent strain of influenza virus can be specifically detected by a simple method. Thus, the present invention is useful for a rapid and simple diagnosis of the diseases of humans, birds and the like caused by such a virus.

### BRIEF DESCRIPTION OF DRAWINGS

Figure 1a is a plan view showing a specific example of the immunochromatographic assay device of the present invention with a single immunochromatographic test strip, and Figure 1b is a longitudinal view in section of the device shown in Figure 1a;
Figure 2 is a plan view showing a specific example of the immunochromatographic assay device of the present invention with two immunochromatographic test strips;
Figure 3 is a plan view showing another specific example of the immunochromatographic assay device of the present invention with two immunochromatographic test strips; and
Figure 4a is a plan view showing a further specific example of the immunochromatographic assay device of the present invention with two immunochromatographic test strips, and Figure 4b is a longitudinal view in section of the device shown in Figure 4a.

### DESCRIPTION OF SYMBOLS

- 1: Adhesive sheet
- 2: Impregnated member
- 3: Membrane carrier
- 4: Capturing zone
- 5: Absorbing member
- 6: Sample-receiving member
- 10: Immunochromatographic test strip
- 20: Immunochromatographic test strip

## Claims

1. A method for detecting a virulent strain of influenza A virus, which comprises providing a first antibody that is reactive with all influenza A virus subtypes and a second antibody that is not reactive with a virulent strain of influenza A virus but is reactive with all influenza A virus subtypes other than the virulent strain, and conducting an immunoassay to detect an antigen that shows a positive response in a reaction with the first antibody and shows a negative response in a reaction with the second antibody.

2. The detection method according to claim 1, wherein the immunoassay comprises a sandwich immunoassay using the first antibody and a third antibody that is reactive with an antigen with which the first antibody is reactive, and/or a sandwich immunoassay using the second antibody and a fourth antibody that is reactive with an antigen with which the second antibody is reactive.

3. The detection method according to claim 2, wherein the first antibody and the second or fourth antibody are immobilized in a carrier.

4. The detection method according to claim 3, wherein the third antibody is reactive with all influenza A virus subtypes and the fourth antibody is not reactive with a virulent strain of influenza A virus but is reactive with all influenza A virus subtypes other than the virulent strain.

5. The detection method according to claim 3, wherein the third and fourth antibodies which may be identical to or different from each other are reactive with all influenza A virus subtypes.

6. The detection method according to any one of claims 1 to 5, wherein the first, second, third and fourth antibodies are monoclonal antibodies against a nucleoprotein of influenza virus.

7. The detection method according to claim 6, wherein the virulent strain of influenza A virus is at least one selected from the group consisting of a subtype H5N1 and virulent strains of subtypes H5N2 and H7N1.

8. The detection method according to claim 7, wherein the virulent strain of influenza A virus is a subtype H5N1.

9. An immunochromatographic assay which comprises:
providing a membrane carrier having a first capturing zone and a second capturing zone which are formed in predetermined positions thereof by immobilizing a first antibody and a second antibody respectively, the first antibody being reactive with all influenza A virus subtypes, and the second antibody being not reactive with a virulent strain of influenza A virus but being reactive with all influenza A virus subtypes other than the virulent strain; and
chromatographically developing a first mixed solution and a second mixed solution in the membrane carrier toward the first capturing zone and the second capturing zone respectively, the first mixed solution containing a test sample and a third antibody reactive with an antigen with which the first antibody is reactive, and the second mixed solution containing the test sample and a fourth antibody reactive with an antigen with which the second antibody is reactive, or chromatographically developing, in the membrane carrier, the first mixed solution toward both the capturing zones,
whereby a virulent strain of influenza A virus can be detected based on a result that a positive response in a reaction with the first antibody is shown in the first capturing zone whilst a negative response in a reaction with the second antibody is shown in the second capturing zone.

10. The assay according to claim 9, wherein the third antibody is reactive with all influenza A virus subtypes, and the fourth antibody is not reactive with a virulent strain of influenza A virus but is reactive with all influenza A virus subtypes other than the virulent strain.

11. The assay according to claim 9, wherein the third and fourth antibodies which may be identical to or different from each other are reactive with all influenza A virus subtypes.

12. The assay according to any one of claims 9 to 11, wherein the first, second, third and fourth antibodies are monoclonal antibodies against a nucleoprotein of influenza virus.

13. The assay according to claim 12, wherein the virulent strain of influenza A virus is at least one selected from the group consisting of a subtype H5N1 and virulent strains of subtypes H5N2 and H7N1.

14. The assay according to claim 13, wherein the virulent strain of influenza A virus is a subtype H5N1.

15. The assay according to claim 9, wherein the third and fourth antibodies are labeled with metallic colloidal particles or latex particles.

16. The assay according to claim 15, wherein the membrane carrier is a nitrocellulose.

17. An immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, a third antibody that is reactive with an antigen with which the first antibody is reactive, a fourth antibody that is reactive with an antigen with which the second antibody is reactive, and a membrane carrier, wherein the first and second antibodies are previously immobilized in predetermined positions of the membrane carrier so as to form a first capturing zone and a second capturing zone respectively, and the third and fourth antibodies are labeled with suitable labeling agents, and wherein the third and fourth antibodies are prepared at a position remote from the capturing zones for being chromatographically developed in the membrane carrier toward the first and second capturing zones, respectively.

18. The assay device according to claim 17, wherein the third antibody is reactive with all influenza A virus subtypes and the fourth antibody is not reactive with a virulent strain of influenza A virus but is reactive with all influenza A virus subtypes other than the virulent strain.

19. The assay device according to claim 17, wherein the third and fourth antibodies which may be identical to or different from each other are reactive with all influenza A virus subtypes.

20. The assay device according to any one of claims 17 to 19, wherein the first, second, third and fourth antibodies are monoclonal antibodies against a nucleoprotein of influenza virus.

21. The assay device according to claim 20, wherein the virulent strain of influenza A virus is at least one selected from the group consisting of a subtype H5N1 and virulent strains of subtypes H5N2 and H7N1.

22. The assay device according to claim 21, wherein the virulent strain of influenza A virus is a subtype H5N1.

23. The assay device according to claim 17, wherein the membrane carrier is a single membrane carrier having the first capturing zone and the second capturing zone that are apart from each other.

24. The assay device according to claim 17, wherein the membrane carrier comprises a first membrane carrier in which the first antibody is immobilized and a second membrane carrier in which the second antibody is immobilized.

25. The assay device according to claim 24, wherein the third antibody is prepared on the first membrane carrier and the fourth antibody is prepared on the second membrane carrier.

26. The assay device according to claim 25, wherein the third and fourth antibodies are prepared whilst being impregnated into members disposed on the first and second membrane carriers, respectively.

27. The assay device according to claim 26, wherein both the first membrane carrier and the second membrane carrier have an elongated form, and the impregnated members disposed on the respective membrane carriers are placed at a certain distance from each other whilst the two membrane carriers are placed in parallel with each other.

28. The assay device according to claim 26, wherein both the first membrane carrier and the second membrane carrier have an elongated form, and the impregnated members disposed on the respective membrane carriers are placed at a certain distance from each other whilst the two membrane carriers are placed radially or oppositely.

29. The assay device according to claim 24, wherein the third and fourth antibodies which may be identical to or different from each other are reactive with all influenza A virus subtypes, and are prepared for being chromatographically developed in the membrane carriers toward the first and second capturing zones.

30. The assay device according to claim 29, wherein the third and fourth antibodies are prepared on the membrane carriers.

31. The assay device according to claim 30, wherein the third and fourth antibodies are prepared whilst being impregnated into members disposed on the membrane carriers.

32. The assay device according to claim 31, wherein the third and fourth antibodies are the same, and the impregnated members are communicated with both the first and second member carriers.

33. The assay device according to claim 32, wherein both the first membrane carrier and the second membrane carrier have an elongated form, and the impregnated members disposed on the respective membrane carriers are placed at a certain distance from each other whilst the two membrane carriers are placed in parallel with each other.

34. The assay device according to claim 32, wherein both the first membrane carrier and the second membrane carrier have an elongated form, and the impregnated members disposed on the respective membrane carriers are placed at a certain distance from each other whilst the two membrane carriers are placed radially or oppositely.

35. The assay device according to claim 17, wherein the third and fourth antibodies are labeled with metallic colloidal particles or latex particles.

36. The assay device according to claim 35, wherein the membrane carrier is a nitrocellulose.

37. The assay device according to claim 17, wherein the membrane carrier is housed in a casing.

38. The assay device according to claim 37, wherein the third antibody and/or the fourth antibody are stored inside or outside the casing.

39. An immunochromatographic assay which comprises:
providing a first antibody that is reactive with all influenza A virus subtypes, a second antibody that is not reactive with a virulent strain of influenza A virus but is reactive with all influenza A virus subtypes other than the virulent strain, and a third antibody that is reactive with an antigen with which the first antibody is reactive;
preparing membrane carriers each having a first capturing zone which is formed in a predetermined position thereof by immobilizing the first antibody; and
chromatographically developing a first mixed solution and a second mixed solution in the respective membrane carriers toward the first capturing zone, the first mixed solution containing a test sample and the second antibody, and the second mixed solution containing the test sample and the third antibody;
whereby a virulent strain of influenza A virus can be detected based on a result that a positive response in a reaction with the third antibody is shown in the first capturing zone whilst a negative response in a reaction with the second antibody is shown in the first capturing zone.

40. The assay according to claim 39, wherein the third antibody is reactive with all influenza A virus subtypes.

41. The assay according to claim 39 or 40, wherein the first, second, and third antibodies are monoclonal antibodies against a nucleoprotein of influenza virus.

42. The assay according to claim 41, wherein the virulent strain of influenza A virus is at least one selected from the group consisting of a subtype H5N1 and virulent strains of subtypes H5N2 and H7N1.

43. The assay according to claim 42, wherein the virulent strain of influenza A virus is a subtype H5N1.

44. The assay according to claim 39, wherein the second and third antibodies are labeled with metallic colloidal particles or latex particles.

45. The assay according to claim 44, wherein the membrane carrier is a nitrocellulose.

46. An immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, a third antibody that is reactive with an antigen with which the first antibody is reactive, and a membrane carrier, wherein the first antibody is previously immobilized in a predetermined position of the membrane carrier so as to form a first capturing zone, and the second and third antibodies are labeled with suitable labeling agents, and wherein the second and third antibodies are each prepared at a position remote from the capturing zone for being chromatographically developed in the membrane carrier toward the first capturing zone.

47. The assay device according to claim 46, wherein the third antibody is reactive with all influenza A virus subtypes.

48. The assay device according to claim 46 or 47, wherein the first, second, and third antibodies are monoclonal antibodies against a nucleoprotein of influenza virus.

49. The assay device according to claim 48, wherein the virulent strain of influenza A virus is at least one selected from the group consisting of a subtype H5N1 and virulent strains of subtypes H5N2 and H7N1.

50. The assay device according to claim 49, wherein the virulent strain of influenza A virus is a subtype H5N1.

51. The assay device according to claim 46, wherein the membrane carrier comprises a first membrane carrier in which the first antibody is immobilized and a second membrane carrier in which the first antibody is immobilized.

52. The assay device according to claim 51, wherein the second antibody is prepared on the first membrane carrier and the third antibody is prepared on the second membrane carrier.

53. The assay device according to claim 52, wherein the second and third antibodies are prepared whilst being impregnated into members disposed on the first and second membrane carriers, respectively.

54. The assay device according to claim 53, wherein both the first membrane carrier and the second membrane carrier have an elongated form, and the impregnated members disposed on the respective membrane carriers are placed at a certain distance from each other whilst the two membrane carriers are placed in parallel with each other.

55. The assay device according to claim 53, wherein both the first membrane carrier and the second membrane carrier have an elongated form, and the impregnated members disposed on the respective membrane carriers are placed at a certain distance from each other whilst the two membrane carriers are placed radially or oppositely.

56. The assay device according to claim 46, wherein the second and third antibodies are labeled with metallic colloidal particles or latex particles.

57. The assay device according to claim 56, wherein the membrane carrier is a nitrocellulose.

58. The assay device according to claim 46, wherein the membrane carrier is housed in a casing.

59. The assay device according to claim 58, wherein the second antibody and/or the third antibody are stored inside or outside the casing(s).

60. An immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, and a membrane carrier, wherein the second antibody is previously immobilized in a predetermined position of the membrane carrier so as to form a first capturing zone, and the first antibody is labeled with a suitable labeling agent, and wherein the first antibody is prepared at a position remote from the first capturing zone for being chromatographically developed in the membrane carrier toward the first capturing zone.

61. An immunochromatographic assay device for detecting a virulent strain of influenza A virus, which comprises at least a first antibody that is reactive with all influenza A virus subtypes, a second antibody that is not reactive with a virulent strain of influenza A virus but is reactive with all influenza A virus subtypes other than the virulent strain, and a membrane carrier, wherein the first antibody is previously immobilized in a predetermined position of the membrane carrier so as to form a first capturing zone, and the second antibody is labeled with a suitable labeling agent, and wherein the second antibody is prepared at a position remote from the first capturing zone for being chromatographically developed in the membrane carrier toward the first capturing zone.

62. A monoclonal antibody which is not reactive with a virulent strain of influenza A virus but is reactive with all influenza A virus subtypes other than the virulent strain.

63. The monoclonal antibody according to claim 62, which is not reactive with a nucleoprotein of a virulent strain of influenza A virus but is reactive with a nucleoprotein of all influenza A virus subtypes other than the virulent strain.

64. The monoclonal antibody according to claim 63, wherein the virulent strain of influenza A virus is at least one selected from the group consisting of a subtype H5N1 and virulent strains of subtypes H5N2 and H7N1.

65. The monoclonal antibody according to claim 64, wherein the virulent strain of influenza A virus is a subtype H5N1.
